# EUROPEAN PATENT APPLICATION

(11) **EP 4 059 561 A1**
(43) Date of publication of application: **21.09.2022**
(21) Application number: 22161481.1
(22) Date of filing: 10.03.2022
(51) Int. Cl.: A61N 1/05, A61N 1/36

(54) **DYSPHAGIA**

(30) Priority: 19.03.2021 GB 202103873
(71) Applicant: Tripathi, Akhil, St Albans AL3 8JH (GB)
(72) Inventor: Tripathi, Akhil, St Albans AL3 8JH (GB)
(74) Representative: HGF

(57) **Abstract**

A mouthpiece (1) for the treatment of dysphagia, the mouthpiece (1) being shaped for receipt and retention within the mouth of a user, the mouthpiece (1) comprises at least one abutment surface for engaging at least one of the inside surface of a lip and/or the inside of a cheek and/or the palate of a user, the abutment surface comprising stimulation means for providing electrical stimulation to the inside surface of a lip and/or the inside of a cheek and/or the palate of a user, in use.

## Description

This invention relates generally to dysphagia mitigation, particularly to devices, methods, systems and control software for dysphagia mitigation. More specifically, although not exclusively, this invention relates to oropharyngeal dysphagia and to its mitigation and to devices, methods, systems and control software for mitigation thereof.

Dysphagia is a condition in which people have difficulties swallowing. This can manifest itself as difficulty with swallowing specific types of foods and/or liquids. Alternatively, in sever cases dysphagia may comprise an inability to swallow at all.

In addition to difficulties in swallowing, the National Health Service (NHS) of the United Kingdom attributes, on its website, the following potential symptoms: coughing or choking when attempting to eat or drink; regurgitation of food or drink (which may be through the nose); the feeling of food or drink being stuck in your chest or throat; drooling of saliva persistently; an inability to properly chew food; and 'wet' sounds generated when attempting to eat or drink. Over time, it is explained, dysphagia may lead to weight loss and/or to repeated chest infections.

It is known, therefore that dysphagia is an unpleasant condition which can be extremely debilitating to those suffering from it. Further, in time, it can lead to further complications which can themselves prove to be serious issues.

Dysphagia can be categorised as either oropharyngeal dysphagia or esophageal dysphagia. Oropharyngeal (or transfer) dysphagia relates to a difficulty in initiating a swallow. Esophageal dysphagia relates to a difficulty in completing a swallow once it has begun. As used herein, the term 'dysphagia' will be taken to refer to oropharyngeal dysphagia and/or esophageal dysphagia.

A number of treatments exist for dysphagia. For example, the form of food and drink may be altered to more readily allow safe swallowing. Alternatively, a feeding tube may be utilized through a person's nose. These treatments, however, do not affect the underlying issue of dysphagia but, instead, merely attempt to lessen the effects of the condition on a person suffering therefrom. As will be appreciated, such treatments also represent a considerable and ongoing imposition on sufferers' ability to enjoy a normal life.

An alternative treatment is surgery to provide a wider oesophagus, for example through stretching the oesophagus or by inserting a tube therein. As will be appreciated, surgery is intrusive and can carry a risk to the health and/or life of the person undergoing such an operation.

Recently a number of treatments have become available. One such treatment is sold under the trademark Phagenyx (RTM) by Phagenesis of Manchester, UK which consists of a catheter which is inserted into the throat of a patient to deliver stimulation to the pharynx. The treatment is devised to treat neurological oropharyngeal dysphagia which may be caused by stroke. This treatment is currently available for sale in the EEA and Switzerland. Another treatment is sold under the trademark Vitalstim (RTM) by Vitalstim UK Limited of Stafford, UK which consists of pads which are attached to the anterior of the neck and provides neuromuscular stimulation (NMES) therapy to seek to treat the condition.

US2007/156182 (Castel) discloses a treatment for dysphagia which comprises one or more channels of electrodes each of which includes a first electrode positioned in electrical contact with tissue of a target region of a patient and a second electrode positioned in electrical contact with tissue of a posterior neck region or a posterior thoracic region of the patient. A series of pulses are then applied to the patient through the one or more channels of electrodes.

It will be appreciated that both commercial treatments stimulate the pharynx, one interiorly (Phagenyx) the other exteriorly (Vitalstim). Whilst treatments for dysphagia are welcome the above commercial methodologies may suffer from compliance issues and, in the case of the Phagenyx treatment at least, are invasive. Further, Castel requires precise positioning of the electrodes to target specific muscles or muscle groups.

It is a non-exclusive object of the invention to provide a device which mitigates dysphagia, which at least partially mitigates one or more of the above-described problems with the prior art devices and/or methods, for example which is less invasive and/or leads to greater compliance and/or which is easier to use.

Accordingly, a first aspect of the invention provides a mouthpiece for treatment of dysphagia (e.g. oropharyngeal dysphagia), the mouthpiece comprising stimulation means (e.g. mechanism) for providing electrical stimulation to the inside of a lip and/or the inside of a cheek of a user, in use.

Advantageously, providing electrical stimulation to the inside of the lip and/or cheek of a user has been found to mitigate dysphagia.

The following features apply to any aspect of the invention.

The mouthpiece may be configured to provide, in use, electrical stimulation to the inside of the lip and/or the inside of the cheek of a user.

The stimulation means may comprise a first electrode means (e.g. first electrode or electrodes), for example which is arranged or arrangeable to apply, in use, an electric current to the inside of the lip of the user.

The stimulation means may comprise a second electrode means (e.g. second electrode or electrodes), for example which is arranged or arrangeable to apply, in use, an electric current to the inside of the cheek of the user.

The mouthpiece may comprise one or more abutment surfaces, e.g. for engaging one or both of the inside of the lip and the inside of the cheek of the user. In embodiments, the mouthpiece may comprise two abutment surfaces for engaging the inside of the cheek of the user. In embodiments, the mouthpiece may comprise (additionally or alternatively) one or more (e.g. two, three or four) abutments surfaces for engaging the inside of the lip of the user.

The mouthpiece may comprise a lip-engaging member, for example which may comprise the or a abutment surface for engaging the inside of the lip (where provided) The first electrode means (where provided) may be operable to electrically stimulate the inside of the lip of the user via the abutment surface. The first electrode means may comprise a first electrode set. The first electrode set may comprise a first electrode and a first counter electrode. The lip-engaging member may be configured to at least partially cover the inside of the lip of the user, in use. The lip-engaging member may be configured to at least partially cover the inside and the outside of the lip of the user, in use. The lip-engaging member may comprise a U-shape profile in cross-section. The lip-engaging member may be configured

(e.g. sized and/or shaped) to fit (e.g. securely) over or onto the lip of the user. The lip-engaging member may have a length, for example which may be greater (e.g. substantially) than its height and/or width. The lip-engaging member may be elongate. The lip-engaging member may be or define a curve. The lip-engaging member may be shaped to conform (e.g. at least partially) to the curve of the user's lip.

The mouthpiece may comprise one or more (e.g. a) cheek-engaging member, for example which may comprise the or a abutment surface for engaging the inside of the cheek (where provided). The second electrode means (where provided) may be operable to electrically stimulate the inside of the cheek of the user via the abutment surface. The second electrode means may comprise a second electrode set. The second electrode set may comprise a second electrode and a second counter electrode. The, some or each cheek-engaging member may comprise one or more substantially planar members (for example two substantially planar members). The abutment surface of the or each cheek-engaging member may be configured (e.g. sized and/or shaped) to conform to the shape of the inside of the cheek of the user.

In embodiments, the mouthpiece may comprise attachment means for attaching the, some or each cheek-engaging member (where provided) to the inside of the cheek of the user, in use.

The mouthpiece may comprise one or more external abutment surfaces, e.g. for engaging one or both of the exterior of the lip and the exterior of the cheek of the user. The one or more external abutment surfaces may be provided in addition or alternative to the abutment surfaces (where provided). In embodiments, the mouthpiece may comprise one or more external cheek-engaging members, for example which may comprise the, some or each external abutment surface. In embodiments, the mouthpiece may comprise one or more external lip-engaging members, for example which may comprise the, some or each external abutment surface. The mouthpiece may comprise further electrode means (e.g. electrode or electrodes). The further electrode means may be operable, in use, to provide electrical stimulation to one or more of the exterior of a cheek of the user and the exterior of the lip of the user. The one or more external cheek-engaging members may comprise the, one, some or each of the one or more external abutment surfaces. The one or more external lip-engaging members may comprise the, one, some or each of the one or more external abutment surfaces.

The mouthpiece may comprise a lip member, for example which may comprise the lip-engaging member (where provided) and the external lip-engaging member (where provided). The lip-engaging member and the external lip-engaging member may be integrally formed (e.g. at least partially).

The mouthpiece may comprise one or more (e.g. a) palate-engaging members and/or one or more tongue-engaging members, for example which may comprise the, some or a abutment surface(s) for engaging the palate or tongue (where provided).

The stimulation means may be configured, in use, to provide electrical stimulation to one or more palate and/or tongue muscles in the user's mouth. The stimulation means may be configured, in use, to provide electrical stimulation to one or both of the upper surface of the tongue and the lower surface of the tongue of the user. Where the upper surface of the tongue is described herein this may be taken to refer to the dorsal or top surface of the tongue. Where the lower surface of the tongue is described herein this may be taken to refer to the sublingual surface of the tongue.

The stimulation means may comprise a third electrode means (e.g. third electrode or electrodes), for example operable to provide electrical stimulation to the upper surface of the tongue of the user, in use, via abutment surfaces. The third electrode means may comprise a third electrode set. The third electrode set may comprise a third electrode and a third counter electrode.

In embodiments, the stimulation means may comprise a fourth electrode means (e.g. fourth electrode or electrodes), for example operable to provide electrical stimulation to the lower surface of the tongue of the user, in use, via abutment surfaces. The fourth electrode means may comprise a fourth electrode set. The fourth electrode set may comprise a fourth electrode and a fourth counter electrode.

The mouthpiece may comprise first and second tongue-engaging members, for example joined together (say at or near one end). The first and second tongue-engaging members may be diverging from one another, say to provide a space therebetween (for example, configured to receive the tongue of a user, in use). The first tongue-engaging members may comprise the third electrode means (where provided) The first tongue engaging members may comprise a first tongue abutment surface and a second tongue abutment surface, each abutment surface may comprise third electrode means. One of the first tongue abutment surface and the second tongue abutment surface may comprise the third electrode and the other may comprise the third counter electrode. The second tongue-engaging member may comprise the fourth electrode means (where provided). The second tongue engaging members may comprise a first tongue abutment surface and a second tongue abutment surface, each abutment surface may comprise fourth electrode means. One of the second tongue abutment surface and the first tongue abutment surface may comprise the fourth electrode and the other may comprise the fourth counter electrode.

Whilst the electrical stimulation may be 'intra' an electrode set, say between the cheek electrode means (*e.g.* the cheek electrode set), it may also be 'inter' electrode sets (for example between the cheek electrode means and the lip electrode means, for example from or between the first electrode of the cheek electrode set to the counter electrode of the lip electrode set, or from the first electrode of the tongue electrode set and the counter electrode of the palate electrode set for instance). Different treatment modalities may require intra and/or inter stimulation, some modalities may combine intra and inter stimulation. However, and whilst it is possible to operably connect exterior electrodes (one which are not operating within the mouth) it is a feature of the invention that the applied stimulation is between electrodes of the mouthpiece and requires no further electrodes mounted outside of the mouth of the user.

At least a portion of the mouthpiece may be shaped and/or sized for receipt within the mouth of the user, in use.

At least a portion of the mouthpiece (e.g. the one or more cheek-engaging member, the lip-engaging member, the first tongue-engaging member and/or the second tongue-engaging member, where provided) may be configured (e.g. sized and/or shaped) to be at least partially held and retained in desired location within a user's mouth by at least a part of the anatomy of the user's mouth. For example, at least a portion (e.g. the one or more cheek-engaging member, the lip-engaging member, the first tongue-engaging member and/or the second tongue-engaging member) of the mouthpiece may be configured (e.g. sized and/or shaped) to be grasped or held between the upper and lower teeth of the user, in use. Advantageously, by providing the electrodes on the mouthpiece, a patient or user can install the mouthpiece and be certain that the correct muscle groups will be stimulated.

The mouthpiece may be configured to provide a current, for example an electric current or impulse current, which may be selected from one or more of a Russian current, interferential current, premodulated current, DC electric current, biphasic electric current triphasic electric current or impulse current. Other current forms may be used. We prefer to apply the smallest potential difference and drive the lowest possible current to effect the required stimulation (and/or muscle contraction). Electrical stimulation may be used in combination with optical, thermal and/or vibratory stimuli to reduce the current required.

The current may be a biphasic symmetrical current, but it may additionally or alternatively be a biphasic asymmetrical current that may either be balanced or unbalanced. The mouthpiece or electrical circuitry (where provided) may be configured to provide, in use, via the first, second, third and/or fourth electrode means (where provided) an electric current with a frequency of up to 150 Hz, say between 1, 2, 3, 4 or 5 Hz and 150 Hz, for example between 10 or 15 and 25 or 30 Hz, e.g. a frequency of about 20 Hz.

By providing a biphasic electric current, particularly a biphasic electric impulse current, lip, cheek, tongue and/or palate muscles contributing to the ability to swallow can be stimulated along with the sensory nerves to mitigate dysphagia.

The electric current may comprise a frequency of between say 1 to 10 Hz, say 1 to 9, 8, 7, 6, 5 Hz or between 10 and 140 Hz, for example between 15 and 130 Hz, preferably between 20 and 120 Hz. Preferably, the electric current comprises a frequency of between 20 and 50 Hz and/or between 50 and 120 Hz. Preferably, the electric current comprises a frequency of between 10 and 30 Hz, e.g. between 15 and 25 Hz, say about 20 Hz. In embodiments, the electrical stimulation may comprise a biphasic impulse electrical current having a frequency of between 5 and 150 Hz, say between 20 and 120 Hz, say between 20 and 50 Hz, e.g. between 50 and 120 Hz, for example between 10 or 15 and 25 or 30 Hz, say about 20 Hz.

The current or at least one, e.g. both, of the first and/or second current may comprise an impulse current. The pulse duration of the or each impulse current may be between 50 and 1000 µs, for example between 100 and 900 µs, e.g. between 150 and 800 µs, preferably between 200 and 700 µs. Preferably, the or a further adjustment means or adjuster, e.g. an pulse duration adjustment means or adjuster, of the mouthpiece is provided for adjusting the pulse duration, for example between one of the aforementioned ranges.

The electrical stimulation or current or first and/or second currents may comprise an intensity or current amplitude, which is preferably selected or selectable to provide maximum contraction of the muscles being treated. By way of example, the intensity or amplitude may comprise approximately 10 mA, for example between 1 and 100 mA, such as between 5 and 50 mA, for example between 5 and 15 mA or between 7 and 25 mA, e.g. between 8 and 12 mA. The or a further adjustment means or adjuster, e.g. an intensity or amplitude adjustment means or adjuster, may be provided for adjusting the intensity or amplitude, for example from 0 to 500 mA or from 0 to 250 mA or from 0 to 200 mA or from 0 to 150 mA or from 0 to 100 mA.

The electric current applied by the first electrode means, the second electrode means, the third electrode means and the fourth electrode means may be the same or different. In embodiments, the fourth electrode means may generate a larger current than the third electrode means. In embodiments, the third electrode means may generate a larger current than the fourth electrode means.

The mouthpiece may comprise electrical circuitry.

The mouthpiece or electrical circuitry may be configured to provide two or more currents, for example a first current and/or a second current and/or a third current, which second current may be different from and/or configurable or settable independently from the first current and/or third current. At least one, e.g. all, of the first and/or second current and/or third current may comprise a biphasic current or a monophasic current, each of which is preferably symmetrical, but may be asymmetrical and either balanced or unbalanced. At least one of the first and/or second current and/or a third current may comprise a frequency of up to 150Hz, say between 5 and 150 Hz, for example between 1 and 150 Hz or between 10 and 140 Hz, e.g. between 15 and 130 Hz, preferably between 20 and 120 Hz. In some embodiments, one or the currents may comprise a frequency of between 1 and 50 Hz (*e.g.* between 1 and 5 Hz), for example 5 to 20 Hz or 20 and 50 Hz and/or the other current may comprise a frequency of between 1 and 120 Hz.

The mouthpiece and/or controller therefore may comprise an adjustment means or adjuster, e.g. a frequency adjustment means or adjuster, for adjusting the frequency of the current or of the first and/or second currents, for example a respective first and second current frequency adjustment means or adjuster for adjusting the frequency of the current, e.g. between one of the aforementioned ranges. The adjustment means or adjuster may be a step-wise adjustment means or adjuster and/or is configured to enable a user to select from one of two or more, e.g. three, four or five, predetermined frequency settings. The mouthpiece may be operable or configured to provide the first and second currents simultaneously and/or concurrently and/or in parallel. Additionally or alternatively, the mouthpiece may be operable or configured to provide the first and second currents in series and/or in sequence and/or in succession.

The mouthpiece and/or a controller therefore may comprise an input means or activator, which may include one or more input devices, buttons and/or push buttons and/or switches and/or dials or the like, e.g. for enabling or activating or initiating the electrical stimulation or current. The mouthpiece may comprise a power source and/or may be configured to be connectable to a power source.

The mouthpiece may comprise or be operably connected to a control means (or controller), for example which may be programmed or programmable, for example to control one or more features of the electrical stimulation or current or currents, for example according to a predetermined treatment regime. The control means may comprise a control system and/or a controller and/or may comprise or be at least partially comprised in the electrical circuitry (where provided). The control means may comprise at least part of the adjustment means or adjuster, e.g. one or more of the frequency and/or pulse duration and/or intensity and/or amplitude and/or treatment duration adjustment means or adjusters.

The input means may be for or configured or operable to control and/or adjust one or more features of the electrical stimulation or electric current or the first and/or second electric current, for example the frequency and/or pulse duration and/or intensity and/or amplitude and/or treatment duration. Additionally or alternatively the mouthpiece or input means may comprise an interface or connection means such as a connector or receptacle for connecting the mouthpiece or control means to another device, such as a personal computer or a handheld device, which may be operable to program and/or control and/or adjust one or more of the aforementioned features.

The mouthpiece or control means or adjustment means may be configured or programmed to control one or more features of the electrical stimulation or electric current or the first and/or second electric current, for example in a predetermined manner and/or according to a predetermined treatment regime. The mouthpiece or control means may be operable or programmable to create and/or alter the predetermined treatment regime, for example by a device to which the mouthpiece is connected, e.g. via the input means.

A further aspect of the invention provides a mouthpiece for treatment of dysphagia, the mouthpiece having at least a portion shaped for receipt within a mouth of a user and comprising an abutment surface for engaging one of the inside of the lip or the inside of the cheek of a user, the mouthpiece further comprising an electrode or electrode means operable to electrically stimulate the inside of the lip or the inside of the cheek of the user via the abutment surface.

A yet further aspect of the invention provides a mouthpiece for treatment of dysphagia, the mouthpiece comprising: a first tongue-engaging member configured to engage, in use, the upper surface of the tongue of a user and comprising first electrode means for providing electrical stimulation, in use, to the upper surface of the tongue of the user; a second tongue-engaging member configured to engage, in use, the lower surface of the tongue of the user and comprising second electrode means for providing electrical stimulation, in use, to the lower surface of the tongue of the user, where the first and second tongue-engaging members are joined together at joining ends thereof, and wherein the first and/or the second tongue-engaging member comprises plural arms at the end opposed to the joining end.

A yet further aspect of the invention provides a mouthpiece for treatment of dysphagia, the mouthpiece comprising a first tongue-engaging member configured to engage, in use, the upper surface of the tongue of a user and comprising first electrode means for providing electrical stimulation, in use, to the upper surface of the tongue of the user; a second tongue-engaging member configured to engage, in use, the lower surface of the tongue of the user and comprising second electrode means for providing electrical stimulation, in use, to the lower surface of the tongue of the user, where the first and second tongue-engaging members are joined together at joining ends thereof, and wherein a gripping portion extends from the joining end of the first and/or second tongue-engaging member, the gripping portion being configured to be gripped by the front teeth of the user, in use.

A yet further aspect of the invention provides a mouthpiece for treatment of dysphagia, the mouthpiece comprising a first tongue-engaging member configured to engage, in use, the upper surface of the tongue of a user and comprising first electrode means for providing electrical stimulation, in use, to the upper surface of the tongue of the user; a second tongue-engaging member configured to engage, in use, the lower surface of the tongue of the user and comprising second electrode means for providing electrical stimulation, in use, to the lower surface of the tongue of the user, where the first and second tongue-engaging members are joined together at joining ends thereof, and wherein the first tongue-engaging member is shaped (e.g. configured or arranged) such that it extends, in use, from its joining end over the tip of the tongue and over the upper surface of the tongue of the user.

A yet further aspect of the invention provides a mouthpiece for treatment of dysphagia, the mouthpiece comprising at least a portion shaped for receipt within a mouth of a user and comprising a first abutment surface for engaging the tongue of a user and a second abutment surface for engaging the roof of the mouth and/or at least a portion of the pharynx of the user, the mouthpiece further comprising first electrode means operable to electrically stimulate the tongue via the first abutment surface and second electrode means operable to electrically stimulate the roof of the mouth and/or at least a portion of the pharynx of the user via the second abutment surface.

The mouthpiece may have one or more abutment surfaces for engaging the sublingual tongue surface and/or the dorsal tongue surface. The mouthpiece may comprise one or more abutment surfaces for engaging the roof of the mouth (for example the soft palate) and/or at least a portion of the pharynx.

The mouthpiece may comprise an arm, for example an arm extending from a base portion. The mouthpiece may comprise a pair of arms extending from a base portion. The pair of arms may extend from the base portion in non-parallel relations. The pair of arms may describe an included angle which is acute. The or a abutment surface may be located on the one or both of the arms. The or both abutment surfaces may be located at or proximate a distal portion of the, one or both arms. The or each abutment surface may be located on the upper surface (*i.e.* the intended upper surface) of the, one or each arm so as to engage the roof of the mouth and/or at least a portion of the pharynx.

The or each arm may also comprise a further abutment surface for engaging the dorsal tongue surface.

The mouthpiece may comprise an arm extending from a base portion for engaging the sublingual tongue surface.

The base portion may be located, in use, proximate the lips of the user, the arm or arms, extending towards the throat of the use inboard of the mandibular arches. The arm or arms are shaped and/or configured to extend throat-wards from the base portion between the mandibular arches of the user.

A yet further aspect of the invention provides an apparatus comprising a mouthpiece as described herein. The apparatus may comprise a or the control means, a or the power source and/or a or the input means.

A further aspect of the invention provides a method of treating dysphagia, the method comprising applying electrical stimulation to the inside of a lip and/or the inside of a cheek of a user, for example using a mouthpiece.

The method may comprise applying electrical stimulation to the one or more palate and/or tongue muscles in the user's mouth.

The method may comprise applying electrical stimulation to the upper surface of the user's tongue and/or to the lower surface of the user's tongue.

The method may comprise at least partially inserting the or a mouthpiece into the mouth of the user, for example and applying electrical stimulation to the inside of the lip and/or the inside of the cheek of the user via at least one portion of the mouthpiece.

The method or treatment regime may comprise providing or applying a current, for example an electric current or impulse current, such as a biphasic electric current or impulse current, e.g. to the one or more oral muscles.

The method may comprise providing or applying a first current and/or a second current, which second current may be different from the first current. The current or at least one, e.g. both, of the first and/or second current may comprise an alternating current. The or at least one of the or each current may comprise a frequency of between 1 and 150 Hz, for example between 10 and 140 Hz, e.g. between 15 and 130 Hz, preferably between 20 and 120 Hz, more preferably between 20 and 50 Hz and/or between 50 and 120 Hz. In some embodiments, the first current comprises a frequency of between 20 and 50 Hz and/or the second current comprises a frequency of between 50 and 120 Hz.

The phases of the biphasic current, or the first and second currents, may be applied simultaneously and/or concurrently. In some embodiments, phases of the biphasic current, or the first and second currents, may be applied in series and/or in sequence and/or in succession.

The current or at least one, e.g. both, of the first and/or second current may comprise an impulse current. The pulse duration of the or each impulse current may be between 50 and 1000 µs, for example between 100 and 900 µs, e.g. between 150 and 800 µs, preferably between 200 and 700 µs.

The electrical stimulation or current or first and/or second currents may comprise an intensity or current amplitude, which is preferably selected or selectable to provide maximum contraction of the muscles being treated. By way of example, the intensity or amplitude may comprise approximately 10 mA, for example between 1 and 100 mA, such as between 5 and 50 mA, for example between 5 and 15 mA or between 7 and 25 mA, e.g. between 8 and 12 mA.

The method may comprise providing or applying the electrical stimulation or electric current or the first and/or second electric current for a predetermined period, which may comprise between 1 minute and 3 hours, for example between 5 and 30 minutes, preferably between 10 and 20 minutes.

The electrical stimulation may be applied in one or more than one stimulation sessions per day. The more than on stimulation sessions per day may be the same or different.

A further aspect of the invention provides a computer program element comprising computer readable program code for causing a processor to execute a procedure to implement a method or treatment regime comprising providing electrical stimulation to the inside of a lip and/or the inside of a cheek of a user.

As will be appreciated, the provision of one or more of electrical stimulation to the inner lip surface, the inner cheek surface and/or the palate from one or more members mounted or provided on or with a mouthpiece provides a convenient way for a user to treat dysphagia.

In preferred embodiments, the mouthpiece may comprise two or more of lip engaging member, cheek engaging members, palate engaging members. Optionally tongue engaging members, for example members for engaging the lower and/or upper surfaces of the tongue may be provided. The engaging members comprise electrodes or electrodes sets. Electrical stimulation may be directed intra electrode sets or inter electrode sets. Conveniently a controller is provided to control electrical stimulation.

The mouthpiece may be worn for a short period, say every day, to train the muscles of the mouth and pharynx/larynx to at least partially mitigate the effects of dysphagia.

Within the scope of this application it is expressly intended that the various aspects, embodiments, examples and alternatives set out in the preceding paragraphs, in the claims and/or in the following description and drawings, and in particular the individual features thereof, may be taken independently or in any combination. That is, all embodiments and/or features of any embodiment can be combined in any way and/or combination, unless such features are incompatible. For the avoidance of doubt, the terms "may", "and/or", "e.g.", "for example" and any similar term as used herein should be interpreted as non-limiting such that any feature so-described need not be present. Indeed, any combination of optional features is expressly envisaged without departing from the scope of the invention, whether or not these are expressly claimed. The applicant reserves the right to change any originally filed claim or file any new claim accordingly, including the right to amend any originally filed claim to depend from and/or incorporate any feature of any other claim although not originally claimed in that manner.

Embodiments of the invention will now be described by way of example only with reference to the accompanying drawings in which:
Figure 1 shows a schematic diagram of a mouthpiece according to an embodiment of the invention;
Figure 2 shows a schematic side view of a controller for use with the mouthpiece of Figure 1;
Figure 3 shows a schematic side view of a charging station for use with the mouthpiece shown in Figure 2 and/or the controller shown in Figure 2;
Figure 4 shows a schematic perspective view of a mouthpiece according to an embodiment of the invention;
Figure 5 shows a schematic perspective view of the mouthpiece shown in Figure 4; and
Figure 6 shows a schematic cross-sectional side view of the mouthpiece shown in Figure 4 received within the mouth of a user.

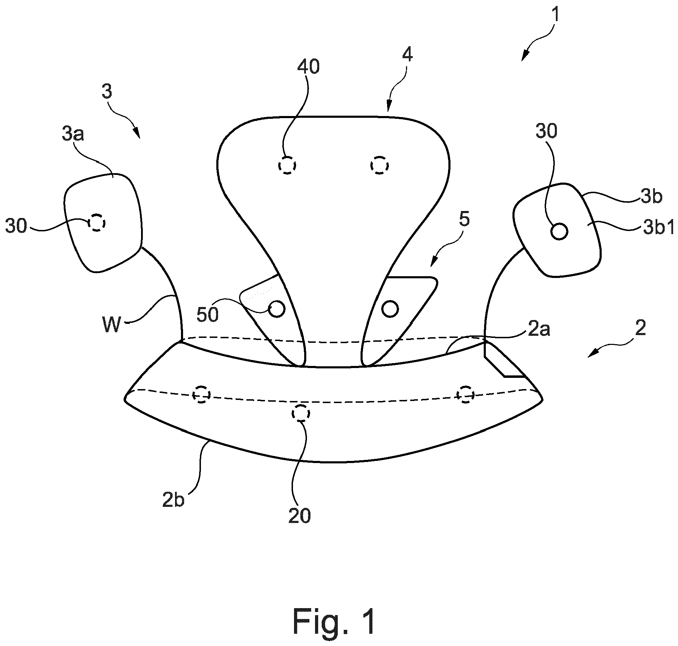

Referring now to Figure 1, there is shown a mouthpiece 1 according to an embodiment of the invention. The mouthpiece 1 comprises a lip member 2, cheek-engaging members 3, a first tongue-engaging member 4 and a second tongue-engaging member 5, in this embodiment.

The mouthpiece 1 (e.g. some or all component parts thereof) may be at least partially formed from a medically acceptable plastic material, for example silicone rubber. At least some of the mouthpiece 1 may be formed from a conductive plastic material, e.g. conductive silicone.

The lip member 2 comprises an internal lip-engaging member 2a and an external lip engaging member 2b. In this embodiment, the internal lip-engaging member 2a and the external lip engaging member 2b are formed integrally. However, in embodiments, they may be separate, joined or joinable together. Additionally or alternatively, the external lip engaging member 2b may not be provided. The lip member 2 (e.g. the internal lip-engaging member 2a) comprises an abutment surface for engaging the inside surface of a lip of a user, in use. The internal lip-engaging member 2a comprises a first electrode set or electrodes 20 (shown as dotted circles in Figure 1). The first electrodes 20 are operable, in use, to electrically stimulate the inside of a lip of a user via the abutment surface, *i.e.* the electrodes 20 may comprise a first electrode and a first counter electrode such that the lip is stimulated by passage of electrical stimulation therebetween. The lip member 2 (e.g. the internal lip-engaging member 2a) is configured (e.g. sized and/or shaped) to fit a lip of a user, in use. In embodiments, at least a portion of the lip member 2 may be formed of a deformable and/or resilient material. The lip member 2 may be configured to at least partially fit onto, over and/or around a lip of a user (e.g. to at least partially cover a lip of a user).

The cheek-engaging members 3 comprise a first cheek-engaging member 3a and a second cheek-engaging member 3b. Each of the first and second cheek-engaging members 3a, 3b comprise an abutment surface 3b1 for engaging the inside of a cheek of a user, in use. Each of the first and second cheek-engaging members 3a, 3b comprise a second electrode set or electrodes 30. The second electrodes 30 are operable, in use, to electrically stimulate the inside of a cheek of a user via the abutment surfaces 3b1. Thus a first cheek engaging member 3a may comprise a second electrode 30 and a second counter electrode 30 such that the cheek may be stimulated by passage of electrical stimulation therebetween. The second cheek engaging member 3b may be similarly arranged. The first and second cheek-engaging members 3a, 3b are configured (e.g. sized and/or shaped) to fit against opposed inside cheeks of a user, in use. The first and second cheek-engaging members 3a, 3b may be or comprise 'pads'. For example, the first and second cheek-engaging members 3a, 3b may comprise first and/or second major surfaces. The first or second major surface may comprise the abutment surface 3b1. The first and second cheek-engaging members 3a, 3b may be at least partially polygonal in shape. The first and second cheek engaging members 3a, 3b may be located, positioned and/or arranged to positively engage the inner surface of the wearer's cheeks.

The first tongue-engaging member 4 comprises an abutment surface for engaging the upper surface of the tongue of a user, in use. The first tongue-engaging member 4 comprises a third electrode set or electrodes 40. The third electrodes 40 are operable, in use, to electrically stimulate the upper surface of the tongue of a user. Thus, a first tongue engaging member 4 may comprise a third electrode 40 and a second counter electrode 40 such that the upper surface of the tongue may be stimulated by passage of electrical stimulation therebetween. The second tongue-engaging member 5 comprises an abutment surface for engaging the lower surface of the tongue of a user, in use. The second tongue-engaging member 5 comprises fourth electrodes 50. The fourth electrodes 50 are operable, in use, to electrically stimulate the lower surface of the tongue of a user. The second tongue engaging member 5 may comprise a third electrode 50 and a second counter electrode 50 such that the lower surface of the tongue may be stimulated by passage of electrical stimulation therebetween.

The first and second tongue-engaging members 4, 5 are joined together at one of their ends. The first and second tongue-engaging members 4, 5 diverge along their lengths. A space is defined between the first and second tongue-engaging members 4, 5. The first and second tongue-engaging members 4, 5 are configured so that the space therebetween is for receipt of part of the tongue of a user.

The mouthpiece 1 may also contains batteries (not shown) and electrical circuitry (not shown). The electrical circuitry may include inverters for converting the direct current of the batteries into currents, for example biphasic currents. The electrical circuitry is connected via wires W to the first, second, third and fourth electrodes 20, 30, 40, 50, in this embodiment.

The mouthpiece 1 may comprise an on/off button (not shown). The mouthpiece 1 may comprise controls (not shown) for operating one or more function thereof. For example, the controls may be used by an operator of the mouthpiece 1 to control the duration, frequency, intensity and/or location of electrical stimulation (e.g. to one/some or each of the electrodes).

In use, the mouthpiece 1 is inserted into the mouth of a user. For example, the first and second cheek-engaging members 3a, 3b are fitted against and/or otherwise attached to the inside of the cheeks of the user, e.g. so that the abutment surfaces 3b1 thereof are engaged with the inside of the cheeks of the user. The lip member 2 is fitted over a lip of the user, for example such that the abutment surface of the internal lip-engaging member 2a of the lip member 2 engages the inside of the lip of the user. The first and second tongue-engaging members 4, 5 are inserted into the mouth of the user so that the tongue of the user is located between the first and second tongue-engaging members 4, 5. In particular, the first tongue-engaging member 4 is positioned such that the abutment surface thereof engages the upper surface of the tongue of the user. The second tongue-engaging member 5 is positioned such that the abutment surface thereof engages the lower surface of the tongue of the user.

The mouthpiece 1 is then turned on (for example via the on/off button). The mouthpiece 1 is then activated to cause electrical stimulation of the inside of the cheek and/or the inside of the lip of the user. Additionally, the mouthpiece 1 may be activated to cause electrical stimulation of the upper and/or lower surface of the tongue of the user.

Two biphasic currents may be applied, each of which is configured with a first set of parameters including intensity, frequency and pulse duration. The parameters may be selected to provide maximal contraction of the muscles of the lip and cheek in the user. The treatment may be carried out for a period of about 60 minutes, for example 10, 20, 30, 40 or 50 minutes.

The intensity, frequency and pulse duration may then be adjusted. The two stimulation signals, *e.g.* biphasic currents may then be applied through the third and/or fourth electrodes 40, 50 to, respectively, the upper or lower surface of the tongue. The second set of parameters may be selected to provide maximal contraction of the user's genioglossus muscle (for example). The treatment may be carried out for a period of about 20 minutes. In embodiments, different sets of parameters may be used for the two biphasic currents supplied to one, some or each of the first, second, third and fourth electrodes 20, 30, 40, 50.

The application of electrical stimulation, *e.g.* biphasic currents, according to the parameters described above stimulate the skeletal muscles of the user's mouth. It is also believed that the application of stimulation, such as biphasic current, to these skeletal muscles creates a further, sensory function, such as a vibratory sensation. Whilst not wishing to be bound by any theory, it is believed that this electrical and vibratory stimulation of the nerves provides feed back to the brain which further enhances the mitigation of dysphagia. Specifically, it is believed that the effectiveness of this treatment is enhanced by multisensory integration within the nervous system.

By way of example, a treatment regime could involve a six week induction period during which each of the aforementioned muscle groups are stimulated for a period of 10 to 20 minutes, twice daily. The treatment regime, which is designed to mitigate dysphagia, could then be followed by an ongoing maintenance regime involving 60 minute sessions once per day.

Whilst the electrical stimulation may be 'intra' an electrode set, say between the first electrode 20 and first counter electrode 20, it may also be inter electrode sets, for example between the first electrode 20 and the second counter electrode 30, or between the first electrode 20 and the third counter electrode 40, or between the third electrode 40 and the first 20 or second 30 counter electrode 40. In all cases, the applied stimulation is between the mouthpiece 1 electrodes and requires no further electrodes mounted outside of the mouth of the user.

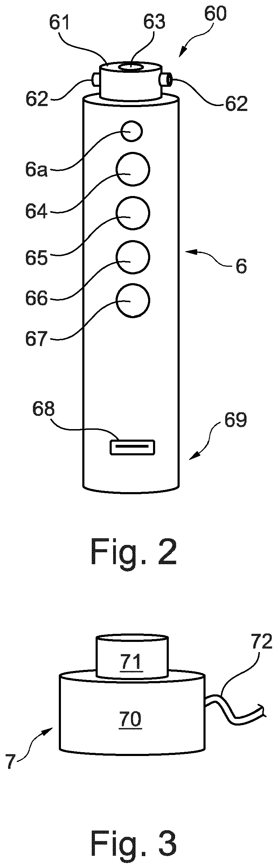

In embodiments, the mouthpiece 1 may be at least partially controlled by a controller 6 (for example, as shown in Figure 2). The controller 6 comprises electrical circuitry (not shown) and batteries (not shown) to power the electrical circuitry, in this embodiment.

The controller 6 is substantially cylindrical in this embodiment and includes a first, upper end 60 with a male connector 61 having a projection 62 on each of its side to provide a bayonet fitting and an electrical socket 63 for receiving an electrical connector (not shown) of the mouthpiece 1. On its front surface, the controller 6 also includes an ON/OFF button 6a, four dials 64, 65, 66, 67, a USB port 68 and a second, lower end 69. In embodiments, the controller 6 may have any suitable shape, and need not be cylindrical.

The mouthpiece 1 may be configured to connect to the controller 6, in this embodiment. For example, the mouthpiece 1 may comprise a socket or receptacle (not shown) for connection of the controller 6. The socket may be provided on or in the lip member 2, the first tongue-engaging member 4 and/or the second tongue-engaging member 5. In embodiments, however, the mouthpiece 1 may be configured to be controlled remotely (e.g. wirelessly for example using a Bluetooth (RTM) connection). In these embodiments, the mouthpiece 1 may not be physically connected or connectable to the controller 6.

The first dial 64 is operable to adjust the current amplitude of, say, a first biphasic current from, say, 0 to 100 mA. The second dial 65 is operable to adjust the current amplitude of , say, a second biphasic current from, say, 0 to 100 mA. The third dial 66 is operable to adjust the duration of the period during which the first biphasic current is supplied from, say, 1 to 30 minutes. The fourth dial 67 is operable to adjust the duration of the period during which the second biphasic current is supplied from, say, 1 to 30 minutes.

The USB port 68 is configured to enable the controller 6 to be charged and/or connected to a personal computer (not shown) or other further electronic device, for example to program one or more characteristics of the first and second biphasic currents independently (and/or to upload or download data to and from the controller). In embodiments, the mouthpiece 1 may comprise a connection (e.g. a USB port or other type of port), for example for programming the first and second biphasic currents and/or for uploading or downloading of data to and from the mouthpiece.

In this embodiment, the frequency of the first biphasic current is set at a value between 2.0 and 50 Hz, the second biphasic current is set at a value between 5.0 and 120Hz and the pulse duration of each biphasic current is set at a value between 200 and 700 µs. The personal computer (not shown) may also incorporate control software operable to override or program the dials 64, 65, 66, 67. The software may be programmed to apply biphasic currents having predetermined characteristics independent from one another, such as amplitudes, frequencies and pulse durations and for a predetermined period of time. It is further envisaged that the mouthpiece 1 and/or the controller 6 could incorporate a memory on which is stored such predetermined characteristics, which may be modified by connecting a personal computer (not shown) or other further electronic device to the mouthpiece 1 or the controller 6 (e.g. via the USB port 28 or a USB port). In such embodiments, the dials may be omitted or configured to adjust the aforementioned characteristics from their pre-programmed values. In some embodiments, it is envisaged that more or less functionality is provided by manual dials, buttons and the like.

In embodiments, the above-described control may be accomplished and/or provided in or by the mouthpiece 1, alone. For example, the electrical circuitry of the mouthpiece 1 may be configured to carry out one, some or each function of control as described above (e.g. regarding the control of biphasic current delivery).

Referring now to Figure 3, there is shown a charging station 7 for charging the controller 6 and/or the mouthpiece 1. The charging station 7 has a cylindrical base 70 with a cylindrical projection 71 and an electrical cable 72 for connecting the charging station 7 to a source of mains electricity. The projection 71 in this embodiment incorporates an inductive coupling and the charging station 7 includes electrical circuitry connected to the electrical mains cable 72 for supplying electricity to the inductive coupling to inductively charge the batteries (not shown) in the mouthpiece 1 and/or the controller 6. The mouthpiece 1 may comprise a receptacle (not shown), for example in the lower end 69. The receptacle of the mouthpiece 1 may be configured to cooperate with the projection 71 of the charging station 7. When connected together the charging station 7 may be operable to charge the batteries in the controller 6 and/or in the mouthpiece 1.

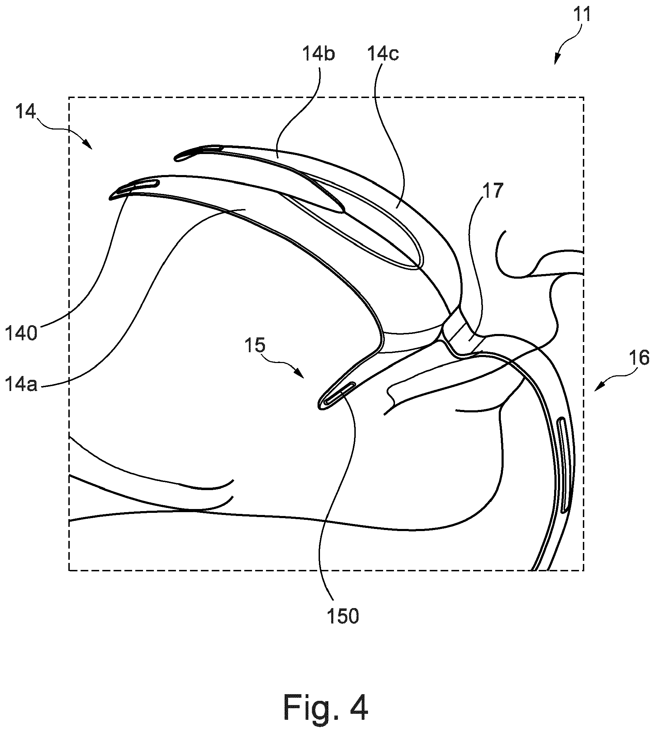

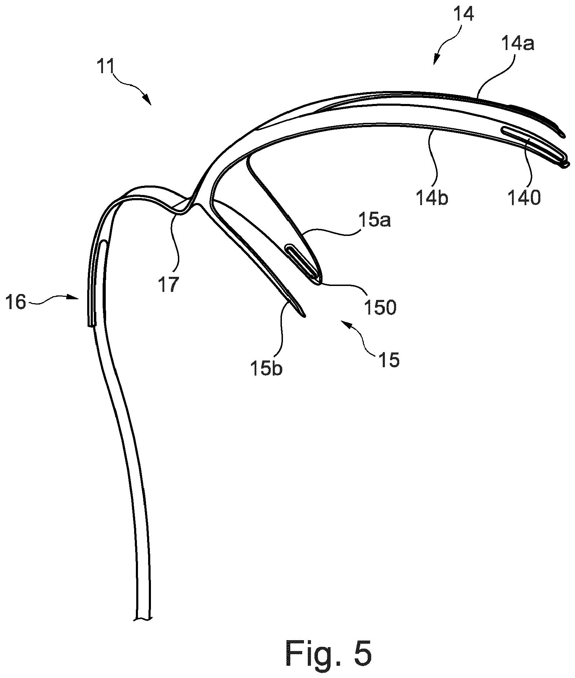

Referring now to Figures 4 and 5, there is shown a mouthpiece 11 comprising first and second members 14, 15 according to an embodiment of the invention. Although not shown, it will be appreciated by one skilled in the art that the mouthpiece 11 comprises a lip-engaging member and cheek-engaging members as described herein (e.g. as shown in Figure 1). In embodiments, however, the mouthpiece 11 may be provided absent a lip-engaging member and/or cheek-engaging members as described herein.

The mouthpiece 11 (e.g. some or all component parts thereof) may be at least partially formed from a body or medically-acceptable plastic material, for example silicone rubber, and at least some of the mouthpiece may be formed from a body or medically-acceptable conductive plastic material e.g. conductive silicone.

The mouthpiece 11 also comprises a controller 16, in this embodiment. The controller 16 may have or comprise electric circuitry and/or a power source (not shown). The controller 16 may comprise a connection for connecting to a source of power, for example a USB connection or the like. The controller 16 may be configured to control operation of the mouthpiece, for example in a manner similar to that described with respect to the mouthpiece 1 shown in Figure 1. The controller 16 may comprise an on/off button. The controller 16 may comprise an input mechanism (such as one or more button or dial or the like) for controlling or altering the electrical stimulation (e.g. one or more parameter such as duration, frequency, amplitude or the like)

The first member 14 is joined to the second member 15 at a joining end thereof. The first member 14 has a 'forked' configuration. The first member 14 comprises first and second arms 14a, 14b. A first end of each of the first and second arms 14a, 14b extend from a common base portion 14c. A second end of each of the first and second arms 14a, 14b is opposed to the first end of each, respective arm 14a, 14b. At or adjacent the second end of each arm 14a, 14b an electrode 140 is provided. The electrodes 140 are arranged to provide electrical stimulation, in use, to the roof of the mouth (palate) of a user and/or to the inside of the throat of the user. The first member 14 is at least partially curved along its length, e.g. from the second ends of the arms 14a, 14b to the base portion 14c. The first member 14 comprises an abutment surface for engaging with the roof of the mouth and/or the inside of the throat of the user, in use. In this embodiment, each of the arms 14a, 14b comprise an abutment surface for engaging with the roof of the mouth and/or the inside of the throat of a user, in use.

The second member 15 comprises first and second arms 15a, 15b (as shown in Figure 5). Each of the arms 15a, 15b extend (e.g. at a first end) from the base portion 14c of the first member 14. In this way, the first and second members 14, 15 are joined together at one of their ends. The second, opposed, end of the arms 15a, 15b of the second tongue-engaging member 15 each comprise an electrode 150. The electrodes 150 are arranged to provide electrical stimulation to the sublingual surface of a user's tongue, in use. The second member 15 comprises an abutment surface for engaging with the sublingual surface of the tongue of a user, in use. In this embodiment, each of the arms 15a, 15b comprise an abutment surface for engaging with the sublingual surface of the tongue of a user, in use.

The second member 15 (e.g. the arms 15a, 15b thereof) extend from the first member 14 at an angle β therefrom. In embodiments, the angle β may be between about 30° and 90°, say between about 35°, 45°, 50°, 55°, 60° and 65°, 70°, 75°, 80°, 85° and 90°.

The second member 15 (e.g. the arms 15a, 15b) is relatively shorter than is the first member 14, in embodiments.

The mouthpiece 11 comprises a gripping portion 17, in this embodiment. The gripping portion 17 extends from the join between the first and second members 14, 15, in this embodiment. The gripping portion 17 is sized and shaped to be gripped between the front teeth of a user, in use. The gripping portion 17 comprises a projection, e.g. from the first member 14 (say from the joining end thereof). The projection is at least partially curved along its length. In embodiments, the curve of the projection is configured to receive the front teeth (e.g. at least some thereof) of the user, when the mouthpiece is in use.

The controller 16 extends from or is joined to the gripping portion 17, in this embodiment.

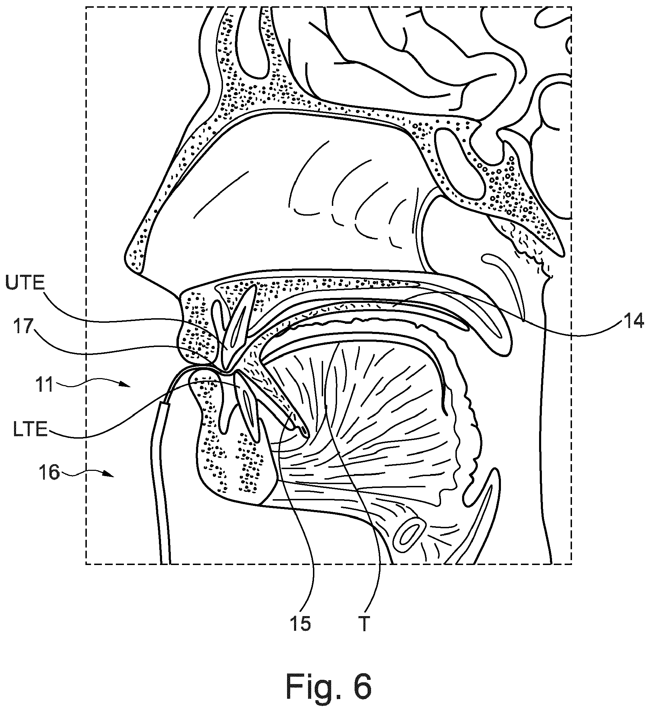

Referring now to Figure 6, the mouthpiece 11 shown in Figures 4 and 5 is shown inserted into the mouth of a user. As can be seen, the second member 15 engages underneath the tongue T of the user. In this way the abutment surfaces of the second member 15 engages the sublingual surface of the user's tongue. The first member 14 extends over the upper (dorsal) surface of the user's tongue T. The abutment surfaces of the first member 14 engage with the roof of the mouth of the user and/or the inside of the throat of the user. The gripping portion 17 is held between the front upper teeth UTE and front lower teeth LTE of the user.

In use, the mouthpiece 11 is activated to provide electrical stimulation via the controller 16 in a similar manner to that described above with respect to the mouthpiece 1. The difference with mouthpiece 11 is that electrical stimulation is applied to the sublingual surface of the tongue and the roof of the mouth and/or the inside of the throat of the user. In embodiments, however, the mouthpiece 11 may be configured to additionally provide electrical stimulation to the upper (dorsal) surface of the tongue of the user. For example, the first member 14 may comprise third electrodes for providing electrical stimulation to the upper (dorsal) surface of the tongue of the user. Where this is the case, the first member 14 may be configured to engage, in use, the upper (dorsal) surface of the user's tongue.

It will be appreciated by those skilled in the art that several variations to the aforementioned embodiments are envisaged without departing from the scope of the invention.

For example, the mouthpiece 1 may take any suitable form, but is designed to enable the electrical stimulation to be applied to the appropriate muscles as described above. Additionally or alternatively, the dials 64, 65, 66, 67 may be replaced with other similar adjustment means or even omitted as explained above. Additionally or alternatively, any suitable number of dials or controls may be provide (and this need not be four, but could instead be 1, 2, 3, 5, 6 or more). Additionally or alternatively, the mouthpiece 1 and controller 6 may be provided as an integral unit. Additionally or alternatively, the charging station 4 may be omitted, for example the controller 6 may comprise a mains cable or connection for charging such as a USB socket or the like or may use single use (primary) batteries or some other power source. Similarly, the USB port 68 may be omitted or replaced with some other connection means, which may be a wireless connection means. Additionally or alternatively, one or both of the tongue-engaging members 4, 5 may be omitted from the mouthpiece 1 or lip and/or cheek engaging members may be added to the mouthpiece 11 for electrically stimulating the lip and/or cheek.

It will also be appreciated by those skilled in the art that any number of combinations of the aforementioned features and/or those shown in the appended drawings provide clear advantages over the prior art and are therefore within the scope of the invention described herein.

## Claims

1. A mouthpiece (1) for the treatment of dysphagia, the mouthpiece (1) being shaped for receipt and retention within the mouth of a user and comprising at least one abutment surface for engaging at least one of the inside surface of a lip and/or the inside of a cheek and/or the palate of a user, the abutment surface comprising stimulation means for providing electrical stimulation to the inside surface of a lip and/or the inside of a cheek and/or the palate of a user, in use.

2. Mouthpiece (1) according to Claim 1, wherein the stimulation means comprises a first electrode set (20) which is arranged or arrangeable to apply, in use, an electric current to the inside of the lip of the user and/or wherein the stimulation means comprises a second electrode set (30) which is arranged or arrangeable to apply, in use, an electric current to the inside of the cheek of the user and/or a further electrode set (140) which is arranged to apply, in use, an electric current to the palate of the user.

3. Mouthpiece (1) according to Claim 2, comprising two or more of the first electrode set (20), second electrode set (30) and further electrode set (140).

4. Mouthpiece (1) according to Claim 2 or 3, wherein the stimulation means is arranged to provide electrical stimulation between electrodes of an electrode set (20, 30, 40) and/or between electrode sets

5. Mouthpiece (1) according to any preceding Claim, wherein the mouthpiece (1) comprises plural abutment surfaces, each comprising stimulation means for providing electrical stimulation to the inside surface of a lip and/or the inside of a cheek and/or the palate of a user, in use.

6. Mouthpiece (1) according to any preceding Claim, comprising a lip-engaging member (2a) which comprises a first of said at least one abutment surface for engaging the inside of the lip.

7. Mouthpiece (1) according to Claim 6, wherein the lip-engaging member (2a) is configured to at least partially cover the inside of the lip of the user, in use and/or wherein the lip-engaging member (2a) is configured to at least partially cover the inside and the outside of the lip of the user, in use.

8. Mouthpiece (1) according to any preceding Claim, comprising at least one cheek engaging members (3a, 3b).

9. Mouthpiece (1) according to Claim 8, wherein said at least one cheek engaging members (3a, 3b), the or each of which comprises at least one of said at least one abutment surface for engaging the inside of the cheek.

10. Mouthpiece (1) according to any preceding Claim, comprising at least one palate-engaging member (14a, 14b), and preferably wherein said at least one palate-engaging member (14a, 14b) comprises at least one of said at least one abutment surface for engaging the palate.

11. Mouthpiece (1) according to any preceding Claim, comprising at least one tongue-engaging member (4, 5; 15a, 15b), and preferably wherein said at least one tongue-engaging member (4, 5; 15a, 15b) comprises at least one of said at least one abutment surface for engaging the tongue of the user, in use.

12. Mouthpiece (1) according to Claim 11, comprising plural tongue-engaging members (4, 5; 15a, 15b).

13. Mouthpiece (1) according to Claim 11 or 12, wherein the or each tongue-engaging member (4, 5;15a, 15b) comprises an electrode set arranged to provide electrical stimulation to the upper surface of the tongue and/or the lower surface of the tongue of the user.

14. Mouthpiece (1) according to any preceding Claim, further comprising control means (16) arranged to control said stimulation means for providing electrical stimulation

15. A dysphagia treatment apparatus comprising a mouthpiece (1) according to any of Claims 1 to 13, and a control means (60), wherein said control means (60) is external to but operatively connected or connectable to said mouthpiece (1).
